# FASCICULE DE BREVET EUROPEEN

(11) **EP 1 296 695 B1**
(45) Date de publication et mention de la délivrance du brevet: **19.05.2004**
(21) Numéro de dépôt: 01951769.7
(22) Date de dépôt: 04.07.2001
(51) Int. Cl.: A61K 35/74, A61P 19/00

(54) **UTILISATION D'UN POLYSACCHARIDE EXCRETE PAR L'ESPECE VIBRIO DIABOLICUS EN CICATRISATION OSSEUSE**
VERWENDUNG EINES POLYSACCHARIDS AUS DEM BAKTERIUM VIBRIO DIABOLICUS ZUR WUNDHEILUNG BEIM KNOCHEN
USE OF A POLYSACCHARIDE EXCRETED BY THE VIBRIO DIABOLICUS SPECIES IN BONE REPAIR

(30) Priorité: 04.07.2000 FR 0008676
(43) Date de publication de la demande: 02.04.2003
(73) Titulaire: IFREMER (INSTITUT FRANCAIS DE RECHERCHE POUR L'EXPLOITATION DE LA MER), 92138 Issy les Moulineaux Cedex (FR)
(72) Inventeur: GUEZENNEC, Jean, F-29280 Plouzane (FR); ZANCHETTA, Philippe, F-29200 Brest (FR); DURAND, Patrick, F-44400 Reze (FR)
(74) Mandataire: Goulard, Sophie
(86) Numéro de dépôt international: PCT/FR2001/002139
(87) Numéro de publication internationale: WO 2002/002051

(56) Documents cités:
- WO-A-98/38327
- ROUGEAUX HELENE ET AL: "Structure of the exopolysaccharide of Vibrio diabolicus isolated from a deep-sea hydrothermal vent" CARBOHYDRATE RESEARCH, ELSEVIER SCIENTIFIC PUBLISHING COMPANY. AMSTERDAM, NL, vol. 322, no. 1-2, 23 novembre 1999 (1999-11-23), pages 40-45, XP002162071 ISSN: 0008-6215
- LOAEC M ET AL: "Chelating properties of bacterial exopolysaccharides from deep-sea hydrothermal vents" CARBOHYDRATE POLYMERS, APPLIED SCIENCE PUBLISHERS, LTD. BARKING, GB, vol. 35, no. 1-2, janvier 1998 (1998-01), pages 65-70, XP002162072 ISSN: 0144-8617
- RAGUENES G ET AL: "VIBRIO DIABOLICUS SP. NOV., A NEW POLYSACCHARIDE-SECRETING ORGANISMISOLATED FROM A DEEP-SEA HYDROTHERMAL VENT POLYCHAETE ANNELID, ALVINELLA POMPEJANA" INTERNATIONAL JOURNAL OF SYSTEMATIC BACTERIOLOGY, SOCIETY FOR GENERAL MICROBIOLOGY, READING, GB, vol. 47, no. 4, 1 octobre 1997 (1997-10-01), pages 989-995, XP002048323 ISSN: 0020-7713

## Description

La présente invention se rapporte à l'utilisation d'un polysaccharide excrété par l'espèce *Vibrio diabolicus* pour la fabrication d'un médicament à activité cicatrisante, ainsi qu'à un biomatériau de reconstruction osseuse comprenant ce polysaccharide.

Les pertes de substance osseuse sont invalidantes et lentes à cicatriser. Lorsque la perte d'os dépasse une dimension critique, il ne peut y avoir de cicatrisation spontanée (J.P. SCHMITZ *et al., Acta Anat.,* 1990, *138*, 185-192). Des matériaux de comblement osseux, qui conduisent ou induisent la cicatrisation, doivent alors être utilisés afin de restaurer la continuité entre les deux berges de la perte osseuse.

Les techniques de greffe les plus anciennes consistent à utiliser de l'os complet, qui comprend, de façon inhérente, des protéines capables d'initier et de favoriser les mécanismes biologiques de la reconstruction osseuse, telles que les BMP ou « Bone Morphogenetic Proteins », l'ostéocalcine, l'ostéopontine et l'ostéogénine. L'autogreffe présente l'avantage d'être de parfaite tolérance immunitaire. Toutefois, le stock osseux est limité chez un individu et l'intervention chirurgicale supplémentaire qui en découle entraîne des risques de complications. Quant aux allogreffes ou xénogreffes, elles présentent des risques de transmission d'agents pathogènes et peuvent entraîner des phénomènes de rejet.

De par les insuffisances des greffes osseuses, les recherches se sont orientées vers des matériaux substitutifs de l'os. Ces matériaux de comblement peuvent être ostéoinducteurs ou ostéoconducteurs.

Les matériaux ostéoinducteurs permettent une régénération de la perte tissulaire selon un mécanisme d'activation cellulaire par les protéines ou peptides du matériau d'apport. Ils sont capables d'induire une ossification en site ectopique, c'est-à-dire extra-osseux. On peut citer, à titre d'exemples de substances et de matériaux ostéoinducteurs autres que l'os sous sa forme native :
- les hormones et les facteurs de croissance (notamment les cytokines, les IGF ou «Insulin-like Growth Factors » et les FGF ou « Fibroblast Growth factors »), dont il a été montré qu'ils favorisent la reconstruction osseuse (P.A. HILL *et al., Endocrinology,* 1995, *136(1)*, 124-131 ; P. CUEVAS *et al., Surg. Neurol.,* 1997, *47*, 242-246; G.L. BARNES *et al., Journal of Bone and Mineral Research*, 1999, *14(11),* 1805-1815). M. ISOBE *et al. (Journal of Biomedical Materials Research*, 1996, *32*, 433-438) ont notamment proposé d'injecter à des rats, par voie sous-cutanée, des facteurs de croissance du type BMP, extraits de tissus osseux. Les BMP, ainsi que, d'une manière générale, les autres facteurs de croissance et les hormones, donnent toutefois des résultats aléatoires lors de leur utilisation en clinique. Leur effet systémique, c'est-à-dire non limité au site d'implantation, présente des risques de diffusion de BMP dans les tissus voisins du site lésé et de calcification locale en site non-osseux. En outre, l'utilisation de BMP peut entraîner des contaminations inter- ou intra-espèces ;
- les dérivés de dextrane portant des fonctions choisies parmi le carboxyméthyle, le benzylamide, le sulfate et le sulfonate (nommés « CMDBS ») et les héparanes sulfates, qui fixent les facteurs de croissance présents au niveau de la lésion en les protégeant des dégradations enzymatiques (D. AVIEZER *et al., The Journal of Biological Chemistry,* 1994, *269(1)*, 114-121 ; F. BLANQUAERT *et al., Journal of Biomedical Materials* Research, 1999, *44,* 63-72 ; M.L. COLOMBIER *et al., Journal de Parodontologie et d'Implantologie Orale,* 1998, *17(4)*, 403-413 ; M. TARDIEU *et al., Journal* of *Cellular Physiology*, 1992, *150,* 194-203). Ces composés nécessitent néanmoins un vecteur, tel que le collagène, pour être utilisés (A. MEDDAHI *et al., Diabetes & Metabolism,* 1996, *22*, 274-278 ; A. MEDDAHI *et al., Path. Res. Pract.*, 1994, *190*, 923-928). A. MEDDAHI *et al*. (1994, *ibid)* ont démontré qu'un dérivé de dextrane particulier, dénommé CMDBS K (comprenant 83% de groupes carboxyméthyles, 23% de groupes benzylamides et 13% de groupes sulfonates), imbibé dans un tampon de collagène permet de combler partiellement, après trois semaines, des défauts osseux de 3 mm de diamètre réalisés dans des calvaria de rats. Ces résultats ne peuvent toutefois pas être extrapolés à des lésions de taille critique, le diamètre critique de référence pour une telle durée étant de 5 à 8 mm.

Par ailleurs, les matériaux ostéoconducteurs, comme leur nom l'indique, ne font que conduire la cicatrisation, sans induire de formation osseuse proprement dite : ils servent de support aux phénomènes biologiques de cicatrisation osseuse et sont destinés à être résorbés au bout d'un certain temps et remplacés par le tissu osseux néoformé. A titre d'exemples de matériaux ostéoconducteurs, on peut citer :
- l'os sous toutes ses formes déprotéinisées et/ou céramisées, d'origine humaine ou bovine par exemple (F.A. PAPAY *et al., The Journal of Craniofacial Surgery*, 1996, *7(5)*, 347-351);
- les phosphates de calcium synthétiques ou naturels tels que le phosphate de calcium tricalcique, le phosphate de calcium biphasique, l'hydroxyapatite, les coraux (F.A. PAPAY *et al., ibid* ; N. B.-A. NAAMAN *et al., The International Journal of Oral & Maxillofacial Implants,* 1998, *13(1)*, 115-120; M. TRECANT *et al., Clinical Materials,* 1994, *15*, 233-240) ;
- les biovitrocéramiques, les bioverres.

Au vu des insuffisances et des inconvénients de cet état de la technique en matière de reconstruction osseuse, les Inventeurs se sont donc donnés pour but de pourvoir à un biomatériau utilisable en reconstruction osseuse, qui soit adapté à la reconstruction de grandes pertes de substances osseuses, c'est-à-dire de défauts dont la dimension est supérieure à la taille critique. Ce biomatériau doit être biocompatible, non-immunogénique et résorbable en fin de reconstruction osseuse.

De façon surprenante, les Inventeurs ont découvert que ces buts sont atteints en utilisant un polysaccharide particulier, à savoir un polysaccharide excrété par l'espèce *Vibrio diabolicus.*

L'invention a pour objet l'utilisation d'un polysaccharide excrété (dit « exopolysaccharide ») par l'espèce *Vibrio diabolicus,* dont la souche a été déposée le 17 octobre 1995 auprès de la CNCM (Collection nationale de Cultures de Microorganismes, 28 rue du Docteur Roux, 75724 Paris, France) sous le numéro 1-1629, pour la fabrication d'un médicament à activité cicatrisante.

Les caractéristiques physico-chimiques et les propriétés métabolites de l'espèce *Vibrio diabolicus* (isolée à partir de la souche HE 800, appartenant au genre Vibrio) sont décrites dans la Demande internationale PCT au nom de l'IFREMER publiée sous le numéro WO 98/38327.

Le médicament à activité cicatrisante défini ci-dessus présente notamment une activité sur la cicatrisation osseuse, par exemple en vue de la préparation d'un matériau de réparation ou de comblement osseux, tels que des implants endo-osseux (implants dentaires et ostéoarticulaires par exemple).

De façon particulièrement avantageuse, le polysaccharide défini ci-dessus peut être utilisé pour la préparation d'un revêtement d'un implant endo-osseux ou d'un matériau de comblement ostéoconducteur. Des matériaux de comblement ostéoconducteurs ou des implants endo-osseux (tels que des prothèses dentaires endo-osseuses en titane ou des prothèses ostéoarticulaires en titane sans ciment orthopédique) revêtus d'un tel polysaccharide peuvent en effet s'intégrer très rapidement dans l'os receveur.

L'invention a également pour objet un implant endo-osseux, caractérisé en ce qu'une partie au moins de sa surface est revêtue d'un polysaccharide tel que défini ci-dessus:

Un polysaccharide utilisable dans l'invention est par exemple celui susceptible d'être obtenu par précipitation à l'éthanol à partir de surnageants de cultures de ladite espèce *Vibrio diabolicus.* Ce polysaccharide est tel qu'il :
- ne comprend pas d'oses neutres,
- présente une teneur en oses acides d'environ 50% en poids,
- présente une teneur en osamines d'environ 50% en poids,
- présente un rapport molaire en monosaccharides acide glucuronique / N-acétyl-galactosamine / N-acétyl-glucosamine d'environ 2/1/1.

La structure d'un tel polysaccharide est exposée en détail dans l'article de H. ROUGEAUX *et al.* paru dans *Carbohydrate Research,* 1999, *322*, 40-45. Il peut être utilisé sous sa forme native ou sous une forme chimique dérivée, le polysaccharide étant par exemple fonctionnalisé par des groupements sulfates.

Le polysaccharide utilisé dans l'invention peut se présenter sous une forme sèche, à savoir sous une forme cotonneuse, fibreuse ou pulvérulente en fonction du traitement final auquel il est soumis (un broyage permet d'obtenir une poudre, une lyophilisation aboutit à un polymère d'apparence cotonneuse et un séchage permet d'obtenir des fibres). Le polysaccharide utilisé dans l'invention peut également se présenter sous une forme hydratée, par exemple sous la forme d'un hydrogel. On pourrait également envisager d'utiliser un tel polysaccharide sous la forme d'une membrane ou d'une structure alvéolée tridimensionnelle solide.

En variante, le polysaccharide utilisé dans l'invention peut être associé à un matériau ostéoconducteur et/ou ostéoinducteur.

Un tel matériau ostéoconducteur peut notamment être sélectionné dans le groupe constitué par l'os déprotéinisé et/ou céramisé, tel que les os commercialisés respectivement par le laboratoire S.P.A.D. (Quetigny, France) et la société OSTE (Clermont-Ferrand, France) sous les dénominations Laddec® et Lubboc® , les phosphates de calcium synthétiques ou naturels (tels que le phosphate de calcium tricalcique de forme β, le phosphate de calcium biphasique, l'hydroxyapatite ou les coraux), les biovitrocéramiques et les bioverres (par exemple le Bioglas® et le Perioglas® commercialisés par le laboratoire PHARMADENT (Levallois-Perret, France).

Quant au matériau ostéoinducteur susceptible d'être associé au polysaccharide utilisé dans l'invention, il peut s'agir d'une hormone ou d'un facteur de croissance, notamment les BMP. L'association dudit polysaccharide à un matériau ostéoinducteur permet d'augmenter la vitesse de la cicatrisation, par exemple par un apport de BMP directement au niveau du site lésé. Le polysaccharide utilisé dans l'invention, excrété par l'espèce *Vibrio diabolicus,* sert de support au matériau ostéoinducteur et le protège des dégradations enzymatiques.

L'invention a également pour objet un biomatériau de reconstruction osseuse, caractérisé en ce qu'il comprend un polysaccharide excrété par l'espèce *Vibrio diabolicus,* dont la souche a été déposée le 17 octobre 1995 auprès de la CNCM sous le numéro 1-1629, ainsi qu'un matériau ostéoconducteur et/ou ostéoinducteur, par exemple tels que ceux décrits ci-dessus.

Outre les dispositions qui précèdent, l'invention comprend encore d'autres dispositions qui ressortiront de la description qui va suivre, qui se réfère à des exemples d'utilisation du polysaccharide produit par la bactérie *Vibrio diabolicus* en cicatrisation osseuse. Il doit être bien entendu, toutefois, que ces exemples sont donnés uniquement à titre d'illustration de l'objet de l'invention, dont ils ne constituent en aucune manière une limitation.

### EXEMPLE 1 : Obtention d'un exopolysaccharide produit par la bactérie Vibrio diabolicus.

### a) Cultures de Vibrio diabolicus.

La souche HE 800 est cultivée sur milieu 2216E (OPPENHEIMER, *J. Mar. Res.,* 1952, *11*, 10-18) enrichi avec du glucose (40 g/l). La production est effectuée à 30°C et à pH 7,4 en fermenteur de 2 litres contenant 1 litre du milieu 2216E-glucosé, tel que décrit par P. VINCENT *et al.* dans *Appl. Environ. Microbiol*., 1994, *60*, 4134-4141. Après environ 48 heures de culture, le milieu de culture présente une viscosité de l'ordre de 100 centipoises à 60 rpm.

### b) Purification de l'exopolysaccharide.

Après dilution de moitié avec de l'eau distillée, les bactéries sont séparées du milieu de culture par une centrifugation à 20 000 g pendant 2 heures. Du chlorure de sodium est alors ajouté à la solution diluée de façon à atteindre une concentration en ce sel de 20 g/l. La solution est maintenue à température ambiante et le polysaccharide est précipité à partir du surnageant à l'aide d'éthanol pur à 4°C. Le polysaccharide est récupéré et subit ensuite plusieurs lavages éthanol/eau en proportions croissantes d'éthanol (70/30, 80/20, 90/10 et 100/0 en volumes), conformément au procédé décrit par F. TALMONT *et al.* (*Food Hydrocolloids,* 1991, *5*, 171-172) ou P. VINCENT *et al. (ibid).*

Le polymère obtenu est séché à 30°C et conservé à température ambiante. Environ 4,5 g de polysaccharide purifié par litre de culture ont ainsi été obtenus.

### EXEMPLE 2 : Utilisation in vivo de l'exopolysaccharide produit par la bactérie Vibrio diabolicus en tant que biomatériau de comblement osseux.

### a) Protocole.

On utilise 10 rats mâles WISTAR âgés de 6 à 7 semaines et pesant de 275 à 299 g. Les rats sont soumis à une anesthésie parentérale à l'aide de Nesdonal® (0,1 ml/100 g, Specia Rhône Poulenc Rorer, Montrouge, France), après une sédation intramusculaire de Kétamine (100 mg/kg) sous formulation Imalgène® (Mérial, Lyon, France) et une prémédication sous-cutanée de Robinul® (0,01 mg/kg, Vétoquinol, Lure, France) une demi-heure avant l'anesthésie.

Des forages de 5 mm de diamètre sont réalisés dans les calvaria des rats, au niveau des deux lobes pariétaux, de part et d'autre de la suture sagittale médiane, selon le protocole décrit par P. CUEVAS *et al. (Surg. Neurol.,* 1997, *47*, 242-6) ou par C. BOSCH *et al. (Cleft Palate Cranofacial Journal,* 1995, *32(4)*, 311-317) : les animaux sont rasés sur le crâne à l'aide d'une tondeuse, puis d'un rasoir bilames. La désinfection pré- et post-opératoire du site chirurgical est faite avec de la Bétadine dermique à 10 % (Asta Medica, Mérignac, France). Une incision médiane est pratiquée sur environ 20 mm et les tissus cutanés sont réclinés, de même que le périoste. Les forages, sous irrigation de sérum physiologique, sont effectués avec des fraises boules diamantées de référence 801.104.014, d'un diamètre de 1,4 mm (Komet, France). Un orifice est pratiqué sur le pariétal droit, un autre sur le pariétal gauche, en prenant soin de ne pas léser la dure-mère.

Une fois les deux forages réalisés, l'exopolysaccharide obtenu dans l'exemple 1 (entré 1 et 2 mg) est positionné, sous sa forme native (structure sèche et d'aspect semblable à du coton), dans la cavité pratiquée dans le pariétal droit, alors que la seconde cavité n'est pas comblée et servira de contrôle. Les sutures périostées sont effectuées avec un fil résorbable Vicryl® 3.0 (Johnson & Johnson Intl., Bruxelles, Belgique) et les sutures des plans cutanés avec un matériau non résorbable.

Les animaux sont ensuite mis dans des cages et sacrifiés par injection de Pentobarbital (Doléthal®,Vétoquinol, Lure, France) au bout de 15 jours. Les calvaria sont prélevées, fixées au formaldéhyde et déminéralisées avant leur étude histologique.

### b) Résultats.

Le polysaccharide excrété par la bactérie *Vibrio diabolicus* permet une cicatrisation osseuse en comblant, pour tous les animaux testés, les cavités réalisées dans les calvaria des rats. Du point de vue histologique, on ne note pas de réactions inflammatoires, le polysaccharide utilisé n'est plus décelable et l'os néoformé en 15 jours est parfaitement structuré : les fibres collagènes sont orientées, les ostéoblastes recouvrent les surfaces osseuses, des ostéocytes sont présents. Cet os est histologiquement normal. On observe également que la néovascularisation est très marquée et que le tissu conjonctif cutané n'a pas proliféré de façon anarchique. En outre, les cicatrices cutanées sont d'excellente qualité, sans phénomène prolifératif.

### c) Expériences comparatives.

Les propriétés cicatrisantes du polysaccharide excrété par la bactérie *Vibrio diabolicus* ont été comparées à trois autres polysaccharides dénommés A, B et C dans le tableau I qui suit.

Le polysaccharide A est un fucane de Phéophycées de haut poids moléculaire (de l'ordre de 1 million de g/mol).

Le polysaccharide B est produit par la souche *d'Alteromonas macleodii* subsp. *fijiensis* dénommée ST 716, décrite par RAGUENES *et al.* dans *Applied and Environmental Microbiology,* 1996, *62(1)*, 67-73 et déposée par l'IFREMER, selon la Traité de Budapest, le 17 octobre 1995 auprès de la CNCM (Collection nationale de Cultures de Microorganismes) tenue par l'Institut Pasteur, 28 rue du Docteur Roux, à Paris, sous le numéro l-1627. L'obtention de ce polysaccharide sous une forme purifiée est décrite dans la Demande internationale PCT publiée sous le numéro WO 99/67411.

Comme décrit dans la Demande internationale PCT publiée sous le numéro WO 99/67411, le polysaccharide B est constitué de glucose, de galactose, d'acide glucuronique, d'acide galacturonique et de mannose pyruvaté, ces différents constituants étant respectivement représentés dans les rapports molaires 1/1/1/2/1, et associés en une unité répétitive hexasaccharidique dans laquelle trois résidus osidiques forment un enchaînement principal, dont le point de branchement est constitué d'un résidu d'acide galacturonique. Sur ce dernier est greffée une chaîne latérale se terminant par un résidu de mannose pyruvaté en position 4 et en position 6.

Un polysaccharide B est constitué de l'unité hexasaccharidique de base répondant à la formule (I) ci-après :

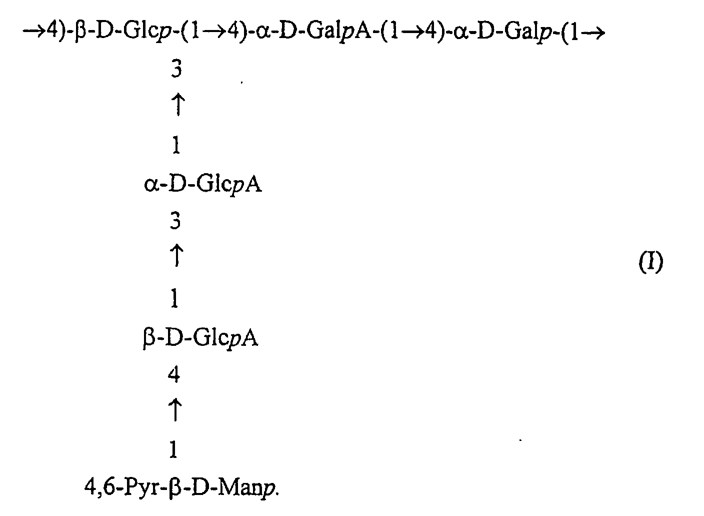

Le polysaccharide C est produit par précipitation, à l'aide d'éthanol à 40% en volume, de surnageants de cultures de la souche de *Pseudoalteromonas* dénommée HYD 721, comme décrit dans l'article de H. ROUGEAUX *et al.* paru dans *Carbohydrate Research,* 1999, *315*, 273-285. L'unité octasaccharidique de base de ce polysaccharide répond à la formule (II) suivante :

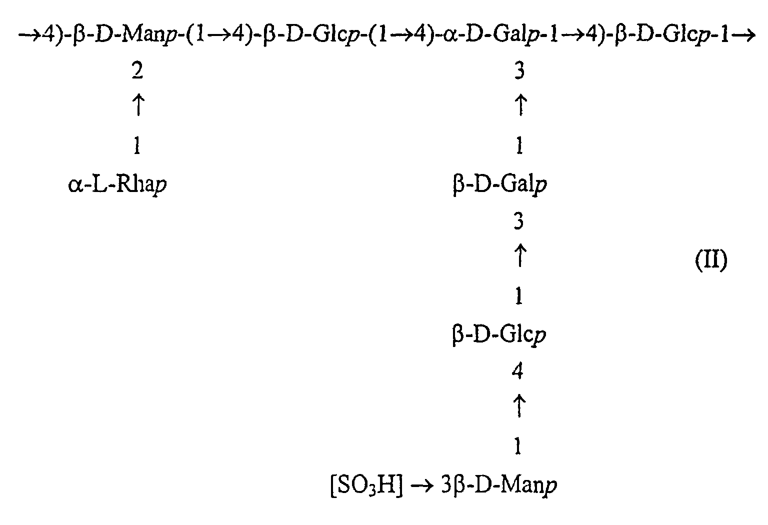

Pour l'appréciation de l'activité cicatrisante des trois polysaccharides A, B et C et de celui excrété par la bactérie *Vibrio diabolicus,* un protocole chirurgical identique à celui décrit au point a) ci-dessus a été utilisé. La quantification des résultats a été réalisée en comparant la superficie de l'os néoformé par rapport à celle des orifices pratiqués à l'origine, comparaison traduite en terme de pourcentage de comblement des orifices. Dans le tableau I ci-dessous figurent donc le nombre d'animaux pour lesquels on observe un comblement des orifices nul (0%), faible (jusqu'à 25%), moyen (jusqu'à 50%), élevé (jusqu'à 99%) ou complet (100%).

**Tableau 1**

| Polymère | Nombre total d'animaux | Comblement des orifices : | | | | |
|---|---|---|---|---|---|---|
| | | nul | faible | moyen | élevé | complet |
| A | 3 | 3 | 0 | 0 | 0 | 0 |
| B | 10 | 0 | 2 | 6 | 2 | 0 |
| C | 5 | 4 | 1 | 0 | 0 | 0 |
| polysaccharide excrété par l'espèce *Vibrio diabolicus* | 8 | 0 | 0 | 0 | 1 | 7 |

Il apparaît donc que, parmi les polysaccharides testés, seul le polysaccharide excrété par la bactérie *Vibrio diabolicus* permet une cicatrisation complète de orifices formés dans les calvaria des rats.

### d) Conclusion.

Ainsi, le polysaccharide excrété par la bactérie *Vibrio diabolicus* permet, de façon reproductible et en 15 jours environ, le comblement de défauts osseux de taille critique (diamètre de 5 mm) dans les calvaria de rats. Des test sur des cavités de 8 mm et avec un laps de temps de 4 semaines ont également permis une cicatrisation complète et de bonne qualité. Les lésions critiquent se reconstituent *ad-integrum* sans les effets incontrôlés observés avec l'utilisation de BMP. Le polysaccharide utilisé n'entraîne aucune réaction inflammatoire et est totalement résorbé par les tissus environnants. Le polysaccharide excrété par la bactérie *Vibrio diabolicus* constitue donc un matériau potentialisateur de la cicatrisation osseuse. Son action est due à ses caractéristiques physico-chimiques originales. Il induit également, à 80% et sur le même intervalle de temps, la cicatrisation de l'orifice témoin.

## Revendications

1. Utilisation d'un polysaccharide excrété par l'espèce *Vibrio diabolicus* pour la fabrication d'un médicament à activité cicatrisante.

2. Utilisation selon la revendication 1, **caractérisée en ce que** ledit médicament présente une activité sur la cicatrisation osseuse.

3. Utilisation selon la revendication 1 ou la revendication 2, **caractérisée en ce que** ledit polysaccharide est utilisé pour la préparation d'un matériau de réparation ou de comblement osseux.

4. Utilisation selon la revendication 1 ou la revendication 2, **caractérisée en ce que** ledit polysaccharide est utilisé pour la préparation d'un revêtement d'un implant endo-osseux ou d'un matériau de comblement ostéoconducteur.

5. Utilisation selon l'une quelconque des revendications précédentes, **caractérisée en ce que** ledit polysaccharide est susceptible d'être obtenu par précipitation à l'éthanol à partir de surnageants de cultures de ladite espèce *Vibrio diabolicus.*

6. Utilisation selon la revendication 5, **caractérisée en ce que** ledit polysaccharide :
- ne comprend pas d'oses neutres,
- présente une teneur en oses acides d'environ 50% en poids,
- présente une teneur en osamines d'environ 50% en poids,
- présente un rapport molaire en monosaccharides acide glucuronique / N-acétyl-galactosamine / N-acétyl-glucosamine d'environ 2/1/1.

7. Utilisation selon l'une quelconque des revendications précédentes, **caractérisée en ce que** ledit polysaccharide est associé à un matériau ostéoconducteur et/ou ostéoinducteur.

8. Utilisation selon la revendication 7, **caractérisée en ce que** ledit matériau ostéoconducteur est sélectionné dans le groupe constitué par l'os déprotéinisé et/ou céramisé, les phosphates de calcium synthétiques ou naturels, les biovitrocéramiques et les bioverres.

9. Biomatériau de reconstruction osseuse, **caractérisé en ce qu'**il comprend un polysaccharide excrété par l'espèce *Vibrio diabolicus,* ainsi qu'un matériau ostéoconducteur et/ou ostéoinducteur.

10. Implant endo-osseux, **caractérisé en ce qu'**une partie au moins de sa surface est revêtue d'un polysaccharide tel que défini dans l'une quelconque des revendications 1 à 6.

## Claims

1. Use of a polysaccharide excreted by the *Vibrio diabolicus* species for the manufacture of a medicament with cicatrizing activity.

2. The use as claimed in claim 1, **characterized in that** said medicament has bone cicatrizing activity.

3. The use as claimed in claim 1 or claim 2, **characterized in that** said polysaccharide is used to prepare a bone repair or filling material.

4. The use as claimed in claim 1 or claim 2, **characterized in that** said polysaccharide is used to prepare a coating for a bone endo-implant or for an osteoconductive filling material.

5. The use as claimed in any one of the preceding claims, **characterized in that** said polysaccharide can be obtained by precipitation with ethanol, from culture supernatants of said *Vibrio diabolicus* species.

6. The use as claimed in claim 5, **characterized in that** said polysaccharide:
- does not comprise any neutral saccharides,
- has an acid saccharide content of approximately 50% by weight,
- has an osamine content of approximately 50% by weight,
- has a glucuronic acid/N-acetylgalactosamine/N-acetylglucosamine monosaccharide molar ratio of approximately 2/1/1.

7. The use as claimed in any one of the preceding claims, **characterized in that** said polysaccharide is combined with an osteoconductive and/or osteoinductive material.

8. The use as claimed in claim 7, **characterized in that** said osteoconductive material is selected from the group consisting of deproteinized and/or ceramized bone, synthetic or natural calcium phosphates, biovitroceramics and bioglasses.

9. Bone reconstruction biomaterial, **characterized in that** it comprises a polysaccharide excreted by the *Vibrio diabolicus* species, and also an osteoconductive and/or osteoinductive material.

10. Bone endo-implant, **characterized in that** at least part of its surface is coated with a polysaccharide as defined in any one of claims 1 to 6.

## Patentansprüche

1. Verwendung eines sezernierten Polysaccharids der Spezies *Vibrio diabolicus* zur Herstellung eines Medikamentes zum Aktivieren der Heilung bzw. Wundheilung.

2. Verwendung nach Anspruch 1, **dadurch gekennzeichnet, dass** das Medikament eine Aktivität bei der Knochenheilung aufweist.

3. Verwendung nach Anspruch 1 oder 2, **dadurch gekennzeichnet, dass** das Polysaccharid zur Herstellung eines Materials für die Wiederherstellung oder für das Auffüllen von Knochen verwendet wird.

4. Verwendung nach Anspruch 1 oder 2, **dadurch gekennzeichnet, dass** das Polysaccharid zur Herstellung von einem Belag eines knocheninternen Implantats oder eines knochenleitenden Füllmaterials verwendet wird.

5. Verwendung nach einem der vorstehenden Ansprüche, **dadurch gekennzeichnet, dass** das Polysaccharid durch Präzipitation mit Ethanol zum Abtrennen von den überstehenden Kulturen der Spezies *Vibrio diabolicus* erhältlich ist.

6. Verwendung nach Anspruch 5, **dadurch gekennzeichnet, dass** das Polysaccharid:
- keine neutralen Monosaccharide umfasst,
- einen Gehalt an sauren Monosacchariden von ungefähr 50 Gew.-% aufweist,
- einen Gehalt an Osaminen von ungefähr 50 Gew.-% aufweist,
- ein molares Verhältnis von sauren Glucoronmonosacchariden/N-Acetylgalactosamin/N-Acetylglucosamin von ungefähr 2/1/1 aufweist.

7. Verwendung nach einem der vorstehenden Ansprüche, **dadurch gekennzeichnet, dass** das Polysaccharid mit einem knochenleitenden oder osteoinduktiven Material verbunden ist.

8. Verwendung nach Anspruch 7, **dadurch gekennzeichnet, dass** das knochenleitende Material ausgewählt ist aus der Gruppe bestehend aus deproteinisiertem und/oder keramisiertem Knochen, synthetischen oder natürlichen Calciumphosphaten, Biovitrokeramiken und Glaskohlenstoffen.

9. Biomaterial zum Wiederaufbau von Knochen, **dadurch gekennzeichnet, dass** es ein sezerniertes Polysaccharid der Spezies *Vibrio diabolicus* umfasst, sowie ein knochenleitendes oder osteoinduktives Material.

10. Knocheninternes Implantat, **dadurch gekennzeichnet, dass** zumindest ein Teil der Oberfläche mit einem Polysaccharid wie dem, das in einem der Ansprüche 1 bis 6 definiert ist, beschichtet ist.
